# EUROPÄISCHE PATENTSCHRIFT

(11) **EP 2 471 004 B1**
(45) Veröffentlichungstag und Bekanntmachung des Hinweises auf die Patenterteilung: **08.08.2018**
(21) Anmeldenummer: 10751771.6
(22) Anmeldetag: 20.08.2010
(51) Int. Cl.: G06F 17/00, A61B 5/04

(54) **VERFAHREN ZUR VERARBEITUNG VON MESSWERTEN EINER DIAGNOSTISCHEN MESSVORRICHTUNG**
METHOD FOR CALIBRATING A DIAGNOSTIC MEASURING DEVICE
PROCÉDÉ D'ÉTALONNAGE D'UN DISPOSITIF DE MESURE DE DIAGNOSTIC

(30) Priorität: 28.08.2009 CH 13362009
(43) Veröffentlichungstag der Anmeldung: 04.07.2012
(73) Patentinhaber: KGmed GmbH, 3532 Zäziwil (CH)
(72) Erfinder: EMESE, Bernhard, 40211 Düsseldorf (DE)
(74) Vertreter: Frei Patent Attorneys
(86) Internationale Anmeldenummer: PCT/CH2010/000203
(87) Internationale Veröffentlichungsnummer: WO 2011/022851

(56) Entgegenhaltungen:
- EP-A2- 1 108 390
- WO-A1-99/36860
- WO-A1-03/057031
- WO-A2-2006/011144
- US-A- 4 587 976
- US-A- 4 700 712
- US-A- 5 749 367
- US-A1- 2002 016 548
- US-A1- 2004 111 021
- US-A1- 2009 088 655
- JAGER F ET AL: "Analysis of transient ST segment changes during ambulatory monitoring", PROCEEDINGS OF THE COMPUTERS IN CARDIOLOGY MEETING. VENICE, SEPT. 23 - 26, 1991; [PROCEEDINGS OF THE COMPUTERS IN CARDIOLOGY MEETING], NEW YORK, IEEE, US, vol. MEETING 18, 23 September 1991 (1991-09-23), pages 453-456, XP010026739, DOI: 10.1109/CIC.1991.169142 ISBN: 978-0-8186-2485-8
- SMRDEL A ET AL: "Advanced detection of st segment episodes in 24-hour ambulatory ecg data by automated tracking of transient st segment reference level", COMPUTERS IN CARDIOLOGY 2002. MEMPHIS, TN, SEPT. 22 - 25, 2002; [COMPUTERS IN CARDIOLOGY], NEW YORK, NY : IEEE, US, vol. VOL. 29, 22 September 2002 (2002-09-22), pages 325-328, XP010624154, DOI: 10.1109/CIC.2002.1166774 ISBN: 978-0-7803-7735-6
- SMRDEL A ET AL: "An algorithm to estimate the ST segment level in 24-hour ambulatory ECG records", COMPUTERS IN CARDIOLOGY, 2008, IEEE, PISCATAWAY, NJ, USA, 14 September 2008 (2008-09-14), pages 701-704, XP031406651, ISBN: 978-1-4244-3706-1

## Beschreibung

Die Erfindung bezieht sich auf ein Verfahren zur Verarbeitung von Messwerten einer diagnostischen Messvorrichtung für biologische Signale, welche als n-dimensionale Vektoren darstellbar sind. In der medizinischen Diagnostik sind solche Messvorrichtungen beispielsweise für die Elektroenzephalographie oder auf allen Gebieten der Kardiographie und der Vektorkardiographie und der Kardiogoniometrie bekannt.

Solche Messvorrichtungen und ihre diagnostische Aussagekraft beruhen zum Beispiel auf der Messung der elektrischen Aktivität eines Organs, wobei sich diese Aktivität im gesunden und kranken Zustand unterscheidet. Im Falle des Herzens beruht die Kardiographie auf einem von Membranströmen der Herzmuskelzellen erzeugten elektrischen Feld. Der (Summen-)Vektor dieses vom Herzen erzeugten elektrischen Feldes ändert in seinem Betrag und in seiner räumlichen Orientierung über die Zeit. Der Herzzyklus, d.h. der elektrische Ablauf eines jeden Herzschlages, kann in verschiedene Abschnitte eingeteilt werden. Im klassischen Elektrokardiogramm entspricht die P-Welle der Vorhoferregung, die R-Welle der Kammer-Depolarisation und die T-Welle der Kammer-Repolarisation.

Bei der in EP 0'086'429 beschriebenen Kardiogoniometrie werden unter Verwendung von vier herznahen thorakalen Elektroden die Herzströme in vier orthogonal zueinander stehenden Projektionen zur Messung der Grösse der Potentiale und deren Ortung im Raum erfasst. Die EP 1'048'000 zeigt eine Weiterentwicklung dieser Lehre auf, welche eine computergestützte rechnerische Analyse zur verbesserten Darstellung und Interpretation der Messresultate führt.

Beispielsweise werden in der räumlichen Darstellung der Vektorkardiographie oder Kardiogoniometrie die oben genannten P-, R- und T-Wellen als P-, R- und T-Vektorschlingen dargestellt. Diese stellen den Weg dar, den die Spitze des Vektors des vom Herzen erzeugten elektrischen Feldes über die Zeit eines Herzschlages durchläuft. Der Summenvektor des vom Herzen erzeugten elektrischen Feldes verläuft zeitlich in drei Schlingen im 3D-Raum. Den Ursprungspunkt des Summenvektors kann man sich als Nullpunkt eines Koordinatensystems für diesen Raum vorstellen. Dieser Nullpunkt muss definiert werden, da je nach Wahl des Nullpunktes unterschiedliche Messwerte entstehen.

Der Nullpunkt sollte einerseits möglichst einem physiologisch begründeten Nullwert entsprechen und andererseits zuverlässig ermittelt werden können trotz der Variabilität der Herzmuskelaktivität auf Grund individueller Unterschiede, Belastungszustand, Gesundheitszustand usw. Ausserdem müssen verschiedene Störeinflüsse wie zum Beispiel Offsetspannungen herausgefiltert werden. Entsprechend schwierig ist es, in einem solchen physiologischen System, einen zuverlässigen Nullpunkt als Referenzpunkt zur Eichung der Messvorrichtung zu finden.

WO 99/36860 beschreibt ein Verfahren zur Vektordarstellung elektrischer Grössen des Herzens und eine elektrische Schnittstelle zur Verarbeitung solcher Grössen. Dabei werden Start- und Endstellen eines räumlichen Signalverlaufes bestimmt, insbesondere eines Nullpunktes, der als Beginn der Depolarisation definiert ist, und des "J-Punktes" zwischen Depolarisation und Repolarisation des Herzens. Dies geschieht, indem von orthogonalisierten, räumlichen Spannungswerten die Beträge ermittelt werden und der zeitliche Verlauf des Betrages differenziert wird. Als Ergebnis wird die zeitliche Lage von Minimumstellen im Signalverlauf des Betrages bestimmt. In dieser Weise bestimmte Minimumstellen können z.B. zum Triggern von Defibrillatoren herangezogen werden.

*"*Analysis Of Transient ST Segment Changes During Ambulatory Monitoring", Jager, F.; Mark, R.G. ; Moody, G.B; Proc. Computers in Cardiology Meeting, 23. Sept 1991, S. 453-456*,* beschreibt die Analyse eines EKG anhand von Messwerten, die zunächst über 16 Herzschläge gemittelt werden, wobei bestimmte Schläge nicht berücksichtigt werden. Anschliessend wird für jede Elektrode eines Zweikanalsystems der isoelektrische Nullpunkt einzeln bestimmt, also ohne, dass eine vektorbasierte Analyse stattfindet.

US 2009/0088655 A1 befasst sich unter anderem mit dem Bestimmen eines Referenzwertes in Form eines mittleren Vektors ("Median isoelectric level"). Dies geschieht jeweils für jeden einzelnen Kanal eines EKG-Signals einzeln. Dabei wird: für jeden Kanal eine Häufigkeitsverteilung der Messwerte durch eine Verteilungsfunktion approximiert und der Wert, bei dem das Maximum der Verteilungsfunktion liegt, als Referenzwertes für diesen Kanal gewählt.

US 4,587,976 beschreibt die Bestimmung von Anfang und Ende eines QRS-Komplexes. Dies geschieht anhand der Projektion des Vektorkardiogrammes auf verschiedene Ebenen. Es wird eine räumliche Geschwindigkeit (durch Differenzbildung der Spannungsvektoren gebildet. Es wird für mehrere räumliche QRS-Schleifen eine Durchschnittsgeschwindigkeit und ein Geschwindigkeitsband um diese berechnet. Eine anschliessende Analyse befasst sich mit der Bestimmung eines Anfangspunktes und eines Endpunktes des QRS-Komplexes.

WO 03/057031 zeigt unterschiedliche Parametrisierungen des Raumvektors der Herzpotentiale, beispielsweise durch räumliche Geschwindigkeit des der Vektorspitze, Punkte minimaler und maximaler räumlicher Geschwindigkeit, Maxima und Mittelwerte der Geschwindigkeit in der T- oder R-Schlinge, J-Punkt, etc. Zum Vergleich kann der Verlauf der Vektorspitze geglättet, orthogonalisiert und normiert werden. Bei der Normierung wird z.B. jede (P, R, T) Schlinge mit ihrem jeweiligen Maximalpotential normiert, so dass alle drei dasselbe Maximalpo-tential aufweisen.

WO 2006/011144 A2 offenbart ein EKG-Messsystem mit Elektroden an nicht gebräuchlichen Stellen des Körpers. Deren Messignale werden in Signale umgerechnet, welche den gebräuchlichen Elektrodenpositionen entsprechen. Dabei wird jeweils für eine einzelne Periode des Herzschlages ein Durchschnittsvektor berechnet. Dies geschieht sowohl für das Messsystem als auch für Standardelektroden, wonach aus mehreren Messungen der beiden Durchschnittsvektoren eine Kompensationstransformation bestimmt wird, welche die Umrechnung von Messungen des neuen Messsystems in Werte entsprechend Standardelektroden erlaubt. Bezugswerte oder Nullwerte für die elektrischen Potentiale werden durch Differenzbildung der elektrischen Signale im Einthoven-Dreieck als Momentanwerte ermittelt und zur Berechnung von Spannungsvektoren der Elektroden (augmented leads) bezüglich des Nullwertes, wiederum als Momentanwerte verwendet.

US 5,749,367 beschreibt die Analyse und den Vergleich von EKG-Signalen mittels "Kohonen Feature Maps" in einem neuronalen Netzwerk. Merkmale eines skalaren EKG-Signales werden extrahiert und bilden einen Merkmalsvektor in einem Merkmalsraum Die Analyse betrifft die Bildung von Clustern und die Klassifizierung von solchen Vektoren.

EP 1 108 390 A2 zeigt eine Vorrichtung zur Analyse von Einzelsignalen oder Merkmalen eines Herzsignales. Dabei wird bei der Detektion eines Ereignisses wie einer Extrasystole ein im zeitlichen Zusammenhang mit dem Ereignis stehender Wert mit einem vorher bestimmten Mittelwert verglichen. Damit lässt sich das Ereignis beurteilen. Als zeitlicher Bezug wird von der Depolarisation oder vom nachfolgenden Nullsignal ausgegangen. Es wird dabei nur eine skalare Mess-grösse eines einzigen Sensors betrachtet.

US 2004/0111021 offenbart ein Verfahren zur Bestimmung eines ischämischen Ereignisses aufgrund des Verlaufes von vektorkardiographischen Signalen. Dabei werden die Signale in den QRS-, ST- und T-Bereichen jeweils mit speziellen Verfahren ausgewertet und die Ergebnisse mit Referenzwerten verglichen. Die Auswertung beinhaltet eine Analyse und Darstellung der 3D-Verläufe der Signale, insbesondere die Bestimmung einer vektoriellen Differenz zu Referenzsignalen (entsprechend einem nicht-ischämischen Zustand), die Bestimmung der zeitlichen Lage des J-Punktes und weiterer charakteristischer Punkte. Im ST-Bereich kann ein Offset festgestellt werden, d.h. dass das Signal nicht zum Nullpunkt zurückkehrt. Dies gilt als Indikator für ein ischämisches Ereignis. Der Offset kann auch in den QRS und T-Bereichen sichtbar sein, und kann somit aus einer Vektordifferenz zu Messungen im gesunden Zustand bestimmt werden. Vorzugsweise wird der Offset unmittelbar nach dem Ende des QRS-Intervalls gemessen. Die Bestimmung von charakteristischen Zeitpunkten im Signalverlauf geschieht anhand des Betrages des Spannungsvektors.

WO 99/36860 beschreibt ein Verfahren zur Vektordarstellung elektrischer Grössen des Herzens und eine elektrische Schnittstelle zur Verarbeitung solcher Grössen. Dabei werden Start- und Endstellen eines räumlichen Signalverlaufes bestimmt, insbesondere eines Nullpunktes, der als Beginn der Depolarisation definiert ist, und des "J-Punktes" zwischen Depolarisation und Repolarisation des Herzens. Dies geschieht, indem von orthogonalisierten, räumlichen Spannungswerten die Beträge ermittelt werden und der zeitliche Verlauf des Betrages differenziert wird. Als Ergebnis wird die zeitliche Lage von Minimumstellen im Signalverlauf des Betrages bestimmt. In dieser Weise bestimmte Minimum¬stellen können z.B. zum Triggern von Defibrillatoren herangezogen werden.

Es ist deshalb die Aufgabe der Erfindung, ein Verfahren zur Eichung einer diagnostischen Messvorrichtung zur Darstellung von biologischen Signalen, insbesondere von biologischen Potentialen, als n-dimensionale Vektoren bereitzustellen, welches diese Schwierigkeit, eine zuverlässige und biologisch möglichst sinnvolle Nullpunktdefinition zu finden, überwindet. Diese Aufgabe wird gelöst durch ein Verfahren wie es im unabhängigen Anspruch definiert ist. Die abhängigen Ansprüche definieren vorteilhafte Ausführungsformen der Erfindung SMRDEL A ET AL: "Advanced detection of ST segment episodes in 24-hour ambulatory ECG data by automated tracking of transient ST segment reference level", [COMPUTERS IN CARDIOLOGY], NEW YORK, NY: IEEE, US, VOL. 29, 22. September 2002, Seiten 325-328*,* zeigt: In jedem Signal eines mehrkanal-EKGs wird einzeln nach einem flachen Segment gesucht und daraus der isoelektrische Referenzpunkt für dieses Signal einzeln ermittelt. Einer von diesen Referenzpunkten wird ausgewählt und bestimmt den Zeitpunkt für einen finalen Referenzpunkt für alle anderen Signale.

SMRDEL A ET AL: "An algorithm to estimate the ST segment level in 24hour ambulatory ECG records", COMPUTERS IN CARDIOLOGY, 2008, IEEE, PISCATAWAY, NJ, USA, 14. September 2008, Seiten 701-704, XP031406651*,* zeigt, was die Bestimmung eines isoelektrischen Nullpunkts betrifft, im Wesentlichen dasselbe Vorgehen wie die vorgenannte Schrift.

Es ist deshalb die Aufgabe der Erfindung, ein Verfahren zur Verarbeitung von Messwerten einer diagnostischen Messvorrichtung zur Darstellung von biologischen Signalen, insbesondere von biologischen Potentialen, als n-dimensionale Vektoren bereitzustellen, welches diese Schwierigkeit, eine zuverlässige und biologisch möglichst sinnvolle Nullpunktdefinition zu finden, überwindet. Diese Aufgabe wird gelöst durch ein Verfahren wie es im unabhängigen Anspruch definiert ist. Die abhängigen Ansprüche definieren vorteilhafte Ausführungsformen der Erfindung Das erfindungsgemässe Verfahren zur Verarbeitung von Messwerten einer diagnostischen Messvorrichtung, betrifft Messvorrichtungen, welche eine Reihe von Messwerten generieren, welche als n-dimensionale Vektoren darstellbar sind. N nimmt einen Wert von mindestens 2 ein. Ein Suchbereich wird definiert für einen in diesem Suchbereich enthaltenen Ruheabschnitt. Der Suchbereich ist definiert als der Bereich, der nach einem Ruheabschnitt abgesucht wird. Der Ruheabschnitt ist ein Abschnitt, in dem der Vektor wenig ändert. Dieser Ruheabschnitt und ein in diesem Ruheabschnitt liegender mittlerer Vektor werden aus im Suchbereich gewonnenen Messwerten ermittelt. Der mittlere Vektor in dem Ruheabschnitt wird als Referenzvektor zur Eichung der Messvorrichtung festgelegt.

In dieser Patentschrift umfasst der Begriff diagnostische Messvorrichtungen nicht nur Messvorrichtungen zur Diagnosestellung, sondern Messvorrichtungen auf allen Gebieten der Human- und Veterinärmedizin, insbesondere auch Messvorrichtungen, welche bei therapeutischen und chirurgischen Anwendungen zum Einsatz kommen.

In einer Ausführungsform entspricht der Suchbereich einem Mittelwert aus mehreren periodisch wiederkehrenden Messwertfolgen.

Es ist ein Grundgedanke der Erfindung, den Nullpunktvektor oder Referenzpunkt (Referenzvektor) für die Eichung der Messvorrichtung als mittleren Vektor zu definieren, der vorzugsweise im Zentrum eines Zeitabschnitts liegt - genannt Ruheabschnitt - indem sich der Vektorbetrag minimal ändert. Ein solcher Ruheabschnitt kann in einer biologisch begründeten Ruhephase liegen wie beispielsweise in einer Ruhephase eines repetitiven physiologischen Zyklus, wie im Falle des oben beschriebenen Herzschlags. Der Ruheabschnitt kann aber auch einer rein empirisch feststellbaren Ruhephase des Signals entsprechen, ohne dass für diese Ruhephase notwendigerweise ursächliche Zusammenhänge bekannt sind. Im Falle einer empirischen Feststellung der Ruhephase wird ein zeitlicher Suchbereich für den Ruheabschnitt bestimmt, welcher eine empirisch feststellbare und/oder physiologisch begründete Ruhephase im Signaloutput enthält. Es kann aber auch die gesamte Zeitspanne der Messung nach einem Ruheabschnitt abgesucht werden.

Als Mass für die Veränderung des Signals dient beispielsweise die Messwertveränderung pro Zeit. Ganz Allgemein wird ein Mass dafür gewonnen, wie sehr in einem Vergleichszeitabschnitt die Messwerte von einem Mittelwert abweichen. Als solches Mass kommt bspw. das arithmetische Mittel der Beträge der Abweichungen in Frage, aber auch die Varianz oder die Standardabweichung oder andere mathematisch sinnvolle Masse für die durchschnittliche Abweichung. Im nachfolgenden Text wird verallgemeinernd der Begriff Messwertveränderung für jedes sinnvolle Mass für die durchschnittliche Abweichung von einem Mittelwert verwendet werden.

Bei der empirischen Feststellung der Ruhephase kann die Veränderung der Messwerte in einem Zeitabschnitt delta verglichen werden mit der Veränderung der Messwerte in anderen Zeitabschnitten und der Zeitabschnitt mit minimaler Messwertveränderung als Ruheabschnitt identifiziert werden. Die Zeitabschnitte delta können als "moving windows" oder als überlappende oder singuläre Zeitabschnitte delta um ausgewählte Messzeitpunkte innerhalb des Zeitverlaufes der Messung gewählt werden. Je nach Anwendung können zur Erfüllung des Kriteriums 'Ruheabschnitt' unterschiedliche Bedingungen an die Veränderung der Messwerte innerhalb eines Zeitabschnittes delta gestellt werden. Solche Kriterien sind beispielsweise, dass die mittlere Abweichung (arithmetisches Mittel der Beträge der Abweichungen, Standardabweichung, Varianz, etc.) vom Mittelwert im Vergleichszeitabschnitt der Messwerte im Ruheabschnitt höchstes ein Drittel so gross, vorzugsweise höchstens halb so gross und besonders bevorzugt mindestens um eine Grössenordnung kleiner als die durchschnittliche Abweichung in Vergleichszeitabschnitten ausserhalb der Ruhephase ist.

Selbstverständlich wird der Fachmann die Vergleichszeitabschnitte oder moving windows sinnvoll so definieren, dass sie der Grösse der zu erwartenden Ruhephase etwa entsprechen, um nicht ruhige und aktive Phasen miteinander auszumitteln. Typischerweise wird die Länge der Vergleichszeitabschnitte so gewählt, dass sie ein Vielfaches der Perioden charakterisischer Signalfluktuationen beinhalten jedoch höchstens einen kleinen Bruchteil (bspw. höchstens 1/10 oder höchstens 1/20) eines ganzen Zyklus betragen. Bei Herzsignalen werden die Vergleichszeitabschnitte beispielsweise bevorzugt eine Länge von zwischen 5 ms und 100 ms, bevorzugt zwischen 10 ms und 50 ms, besonders bevorzugt zwischen 15 ms und 30 ms, bspw. 20 ms haben. Im Allgemeinen Fall (d.h. nicht nur auf Herzsignale bezogen) können die Perioden charakteristischer Signalfluktuationen bspw. durch Fouriertransformation bestimmt werden (die gesuchte Periode entspricht dem Inversen der Frequenz, bei der das Fourierspektrum ein Maximum aufweist), wobei oft das Augenmass der Fachperson die Länge der Vergleichszeitabschnitte besser festlegen kann als ein mathematischer Algorithmus.

Der Suchbereich kann die ganze Messperiode also beispielsweise Messwerte über mindestens einen ganzen Signalzyklus beinhalten oder auch nur einen Teil der Messperiode umfassen. Es ist auch möglich dieses Verfahren zur Auffindung eines Ruheabschnittes zuerst über grössere Zeitabschnitte durchzuführen und innerhalb eines dann identifizierten Ruheabschnittes zu wiederholen, was in einer zunehmenden Verfeinerung des Verfahrens zur Identifizierung des Ruhepunktes führt.

Gemäss einer bevorzugten Ausführungsform kann also in einem beliebigen Suchbereich, in welchem eine Ruhephase vorhanden ist, ein Ruheabschnitt identifiziert werden. Das gilt sowohl, wenn der Suchbereich die ganze Messperiode beinhaltet, als auch wenn sie nur einen Teil der Messperiode umfasst.

Gemäss einer weiteren bevorzugten Ausführungsform wird der Suchbereich für einen Ruheabschnitt nicht den ganzen Zeitverlauf der Messung umfassen, sondern wird auf eine biologisch sinnvolle Zeitspanne innerhalb des ganzen Zeitverlaufs der Messung beschränkt. Ein biologisch begründeter Suchbereich kann beispielsweise aus empirischen Daten, welche physiologische Ruhephasen anzeigen, ermittelt werden und/oder aus dem Wissen über den Ablauf des zu untersuchenden physiologischen Prozesses, insbesondere über dessen Phasen ohne oder mit nur minimaler physiologischer Aktivität. Eine solche Einschränkung des Suchbereichs weist den Ruheabschnitt mit minimaler Messwertveränderung des Signals und den mittleren Vektor im Zentrum dieses Ruheabschnittes in eine physiologische Ruhephase.

Ein grosser Vorteil dieses Verfahrens zur Verarbeitung von Messwerten einer Messvorrichtung ist es, dass es für alle Aufzeichnungen von biologischen Signalen, welche sich als n-dimensionale Vektoren darstellen lassen, anwendbar ist, beispielsweise als zwei- oder drei dimensionale Vektoren oder als mehrkanalige n-Tupel, welche einen Messwert zu einem bestimmten Zeitpunkt repräsentieren. Als Beispiel für eine mehrkanalige Anwendung sei hier das bekannte 12-Kanal-EKG genannt. In der Vektorkardiographie wird der Summenvektor des vom Herzmuskel erzeugten elektrischen Feldes als ein dreidimensionaler Vektor dargestellt.

Am Beispiel der Kardiographie und insbesondere der Vektorkardiographie wird im folgenden das Verfahren zur Eichung einer Messvorrichtung ausführlich beschrieben, wobei dies nicht bedeutet, dass die Erfindung auf kardiographische Anwendungen beschränkt ist.

Ein physiologisch begründeter Nullpunkt, der deshalb als Referenzpunkt zur Eichung einer Messvorrichtung der elektrischen Herzaktivität geeignet ist, befindet sich an dem Ort mit kleinstmöglichem elektrischem Potential, nachdem sich alle Erregung gelegt hat und noch keine neue begonnen hat. Der diese Bedingung erfüllende Nullpunkt als Referenzpunkt zur Eichung wird entsprechend als isoelektrischer Nullpunkt definiert. Stimmte dieser Nullpunkt mit dem isoelektrischen Nullpunkt überein, so würde die Messung des Vektors des vom Herzen erzeugten elektrischen Feldes in einer Ruhephase theoretisch ein Potential von 0 Volt in allen Ableitungen ergeben. Die gemessenen Potentiale der Ableitungen werden durch Störungen des Messsystems allerdings wie eingangs erwähnt verfälscht, so dass selbst zu einem Messzeitpunkt, an dem gerade ein vollkommen isoelektrisches Potenzial anliegt, nie genau 0 Volt in allen Ableitungen gemessen wird. Mit Hilfe des erfindungsgemässen Verfahrens kann der Nullpunkt als Referenzpunkt zur Eichung so ermittelt werden, dass er möglichst gut dem isoelektrischen Nullpunkt entspricht, das heisst, dass ein Potential von 0 V in den Messdaten immer dann in bester Näherung angezeigt wird, wenn ein Ruhepotential am Körper tatsächlich vorhanden war.

Im Verlaufe des Herzzyklus nähert sich der Vektor des vom Herzen erzeugten elektrischen Feldes in mehreren kurzen Ruhephasen jeweils dem theoretisch definierten isoelektrischen Nullpunkt, insbesondere in den Phasen nach der Vorhoferregung (P-Welle) und vor der Kammer-Depolarisation (R-Welle) also kurz vor dem sogenannten Z-Point, sowie nach der Depolarisation und vor der Repolarisation der Kammer (T-Welle) am sogenannten J-Point und nochmals nach der Kammer-Repolarisation und vor der Vorhoferregung des nächstes Schlages. Am bedeutendsten für die Bestimmung des isoelektrischen Nullpunktes ist die Ruhephase vor der Depolarisation der Kammern (R-Welle), weil dann die Fortleitung der elektrischen Aktivität im AV-Knoten ruht und daher der Z-Punkt noch nicht erreicht ist und noch keine Aktionspotentiale von ventrikulären Myokardzellen ablaufen und auch die Repolarisation der Vorhofzellen noch nicht eingesetzt hat.

Weiter liegt der Erfindung eine Beobachtung zugrunde, die bei Aufzeichnungen in Ruhe von ca. 1000 Patienten aus Studien gemacht wurde, nämlich, dass es in nahezu allen Aufzeichnungen von Potenzialverläufen der Patienten einen merklichen Zeitraum zwischen der Vorhoferreguung, (P-Welle/P-Schlinge) und Kammer-Depolarisation (R-Welle/R-Schlinge) gibt, in dem sich der gemessene Vektor des vom Herzen erzeugten elektrischen Feldes kaum verändert. Mehrere Messpunkte hintereinander in allen Ableitungen liefern also konstante Werte, welche sich in dieser Zeitspanne um einen gedachten Mittelpunkt streuen. Diese Zeitspanne mit isoelektrischem Potenzial entspricht also einer Ruhephase mit minimaler Veränderung des Vektors und dauert je nach Person meist zwischen 20 ms und 80 ms oder auch länger.

Da wie oben dargelegt auch die Physiologie des Herzens eine solche Ruhephase vor der Depolarisation vorgibt, wird in bevorzugten Ausführungsformen des Verfahrens der Suchbereich so gewählt, dass er diese Ruhephase einschliesst, aber nicht auf den ganzen zeitlichen Verlauf eines Herzschlages ausgedehnt ist. Er erstreckt beispielsweise vom Potentialmaximum der Vorhoferregung bis zum Maximum der Kammer-Depolarisation. Der Suchbereich kann auch kleiner gewählt werden, so lange sichergestellt ist, dass er die Ruhephase einschliesst. Innerhalb des definierten Suchbereiches wird nun auf der Basis der Rohdaten mit einem numerischen Verfahren der Ruheabschnitt und der in dessen Zentrum stehende mittlere Vektor als Nullpunktvektor oder Referenzvektor ermittelt. Dieses Vorgehen gewährleistet, dass der Nullpunkt als Referenzpunkt zur Eichung in die physiologische Ruhephase vor der Kammer-Depolarisation (R-Welle, R-Schlinge) fällt und in bestmöglicher Näherung dem isoelektrischen Nullpunkt entspricht.

Ein weiterer Vorteil für kardiologische Anwendungen dieses Verfahrens ist, dass selbst wenn die Ruhephase vor der Kammer-Depolarisation nur ganz kurz andauert, was bei einigen Patienten vorkommt, es dennoch möglich ist, einen physiologisch sinnvollen Nullpunkt als Referenzpunkt zur Eichung zu ermitteln. Unter Anwendung eines wie nachfolgend beispielhaft beschriebenen numerischen Verfahrens wird ein Nullpunkt ermittelt, an welchem sich der zeitliche Verlauf des Vektors am wenigsten schnell ändert, also sich am meisten in Ruhe befindet. Das Verfahren ist deshalb wenig fehleranfällig. Mindestvoraussetzung ist nur, dass die R-Welle des Schlages bereits eindeutig lokalisiert wurde, wobei eine ungefähre noch nicht exakte Lokalisation bereits genügen kann.

Ein weiterer Vorteil des Verfahrens ist, dass die angewandte Berechnung eines Nullpunktes als Referenzpunkt auch invariant ist gegenüber einer Rotation und Translation der Messwerte. Sie kann sowohl direkt auf unbearbeitete Samples einer Messung, als auch auf gefilterte oder geglättete oder gemittelte Messungen angewendet werden und liefert in allen Fällen gleich gute Ergebnisse.

Das erfindungsgemässe Verfahren eignet sich auch zur Eichung von Messvorrichtungen ausserhalb der Kardiologie beispielsweise in der Elektroenzephalographie zur Messung der elektrischen Aktivität des Gehirns, wobei analog zum Obigen der Suchbereich für den Referenz-Nullpunkt entsprechend so gewählt werden soll, dass darin ein physiologischer Ruheabschnitt erwartet werden kann. Weitere Anwendungen des erfindungsgemässen Verfahrens betreffen die Eichung von Messvorrichtungen für andere biologische Vorgänge wie hormonelle oder andere chemische oder physikalische Abläufe, bei welchen Messdaten über einen Zeitabschnitt erhoben und auf einen Referenzpunkt bezogen werden.

Ein weiterer Aspekt der Erfindung betrifft eine Messvorrichtung, welche biologische Signale wie beispielsweise Potentiale misst und Mittel aufweist, das Verfahren wie vorstehend beschrieben auszuführen. Eine bevorzugte Ausführungsform einer Messvorrichtung misst Potenzialveränderungen eines menschlichen oder tierischen Organs wie des Herzens oder das Gehirnes mittels Elektroden, bspw. an der Körperoberfläche, indem die Signale abgeleitet und diese Signale mit aus dem Stand der Technik bekannten Geräten oder Gerätekomponenten als Messwerte erfasst, verarbeitet und aufgezeichnet werden. Diese Geräte oder Gerätekomponenten weisen Mittel zur Anwendung des erfindungsgemässen Verfahrens zur Eichung der Messvorrichtung auf. Die Mittel zur Anwendung des erfindungsgemässen Verfahrens können beispielsweise die Programmierung des Gerätes oder einer Gerätekomponente oder eine Softwarekomponente sein.

Beispielhafte Ausführungsformen des Verfahrens werden im Zusammenhang mit den folgenden Figuren mehr im Detail beschrieben.
- **Figur 1**:: Vergrösserter Ausschnitt einer räumlichen Aufzeichnung eines Herzschlages, welcher Vektorschlingen und ruhende Vektorknäuel eines Vektorkardiogramms aufzeigt.
- **Figur 2:**: Flussdiagramm eines beispielhaften Verfahrens zur Ermittlung eines Nullpunktes als Referenzpunkt zur Eichung entsprechend dem isolektrischen Nullpunkt als geometrischer Mittelpunkt eines ruhenden Vektorknäuels.

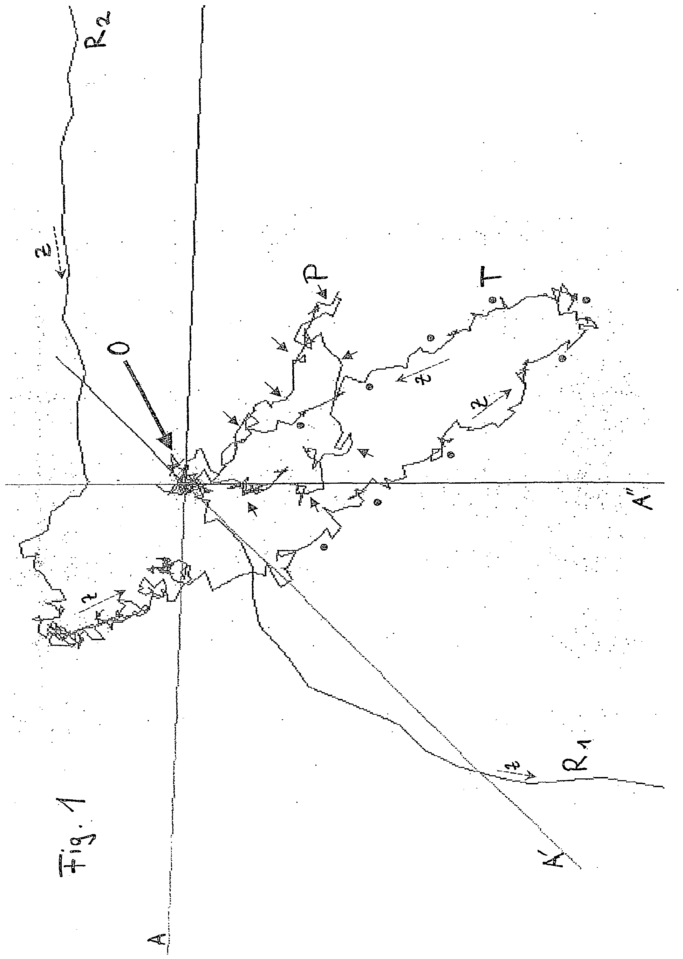

**Figur 1** betrifft die Anwendung des Verfahrens für eine Messvorrichtung zur Vektorkardiographie. Sie zeigt einen vergrösserten Ausschnitt einer räumlichen Darstellung der Herzpotenziale, wobei für jeden Zeitpunkt t der Vektor des vom Herzen erzeugten elektrischen Feldes ermittelt wurde. Das Vektorkardiogramm zeigt die Verbindungslinie der Spitzen dieser Vektoren von einem zum nächsten gemessenen Sample. Jeder Herzzyklus produziert dabei mehrere charakteristische Schlingen, die unterbrochen sind von Phasen, in denen der Vektor (d.h. die Stärke und räumlich Orientierung des elektrischen Felds) relativ konstant verweilen. Eine solche Ruhephase ist in Figur 1 sichtbar wie ein Knäuel von kurzen Linien (d.h. Verbindungslinien von Vektor zu Vektor), die nahezu richtungslos um einen Mittelpunkt O (markiert mit einem langen Pfeil) herumwandern bzw. nur im Microvoltbereich streuen. Vor der hier betrachteten Ruhephase vor der R-Schlinge wandert der Vektor von der P-Schlinge P (markiert mit acht kurzen Pfeilen) kommend in dieses "ruhende Knäuel" hinein, und nach dieser Ruhephase tritt er aus dem Knäuel heraus und verlässt den Nullpunkt sehr schnell und weit, um mit grosser Geschwindigkeit die R-Schlinge zu durchlaufen (R₁ markiert den vom Nullpunkt wegführenden Schenkel der R-Schlinge). Das Ende der R-Schlinge (R₂) und die anschliessende T-Schlinge T (markiert mit Punkten) sind ebenfalls in der Figur dargestellt. Der isoelektrische Nullpunkt O liegt in der geometrischen Mitte des Knäuels vor der R-Schlinge, und der Koordinatenursprung ist in den isoelektrischen Nullpunkt gelegt. Die drei orthogonalen Achsen A, A' und A", die den hier 3D Raum definieren, schneiden sich definitionsgemäss im Nullpunkt. Die gestrichelten lanzettförmigen Pfeile Z entlang der Schlingen geben den zeitlichen Ablauf der Ausbreitung des Feldvektors im Raum an.

Das erfindungsgemässe Verfahren zur Eichung einer diagnostischen Messvorrichtung für biologische Potentiale kann für alle Aufzeichnungsmethoden von Herzpotentialen wie beispielsweise auch für die Elektrokardiographie angewendet werden, und ist nicht auf die in dieser Figur beschriebene Ausführungsform für räumliche Aufzeichnungen beschränkt.

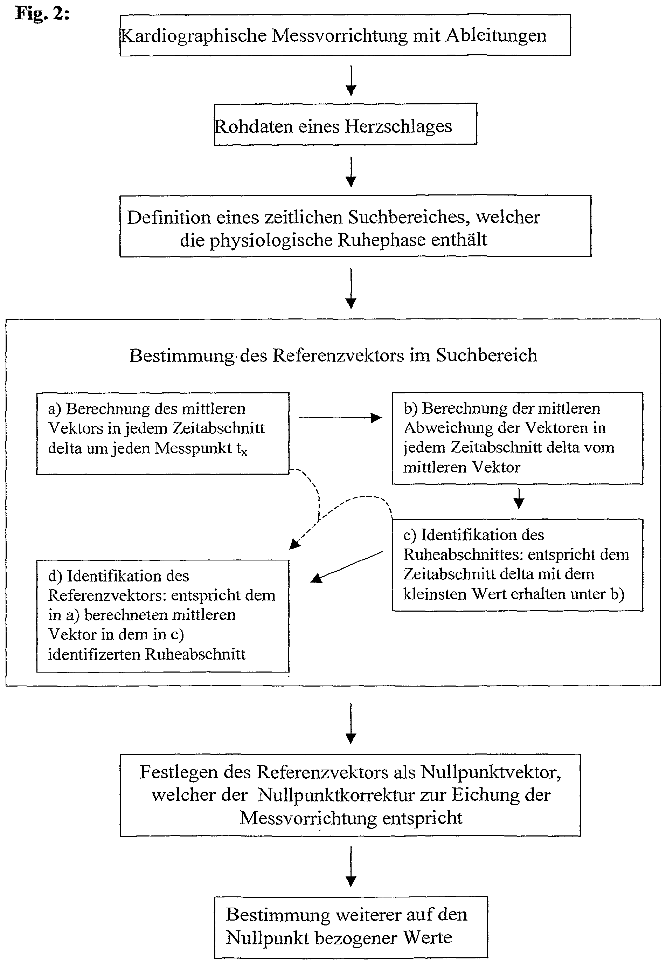

**Figur 2** zeigt ein Flussdiagramm eines beispielhaften Verfahrens zur Eichung einer *vektorkardiographischen Messvorrichtung, wobei der Nullpunkt als Referenzpunkt* zur Eichung als geometrischer Mittelpunkt eines ruhenden Vektorknäuels identifiziert wird.

Zur Eichung einer kardiographischen Messvorrichtung wird aus den Rohdaten eines Herzzyklus zuerst ein zeitlicher Suchbereich definiert, in welchem ein Ruheabschnitt gefunden werden soll. Der Suchbereich muss gross genug sein, um eine physiologische Ruhephase zu beinhalten und erstreckt sich maximal vom Potentialmaximum der Vorhoferregung (Spitze der P-Schlinge) bis zum Potentialmaximum der Kammer-Depolarisation (Spitze der R-Schlinge). Der Suchbereich kann aber auch kleiner gewählt werden, solange gewährleistet ist, dass er einen Ruheabschnitt beinhaltet.

Anschliessend wird eine Anzahl Zeitpunkte tₓ für x=1...n innerhalb dieses Suchbereiches ausgewählt. In Zeitintervallen delta um diese Zeitpunkte tₓ herum wird aus den Messwerten der Signalvektoren der Nullpunktvektor numerisch ermittelt und zur Eichung als Referenzvektor verwendet. Eine mögliche Variante einer solchen numerischen Ermittlung umfasst die Schritte a)- d) aus Abb. 2. Die Erfindung ist nicht beschränkt auf Verfahren, welche eine numerische Ermittlung nach diesem Beispiel anwenden. Je nach Anwendung können beispielsweise folgende Varianten für sich oder - wo möglich - in Kombination berücksichtigt werden:
- Verzicht auf die Auswahl eines Suchbereiches, stattdessen Mittelung über den ganzen Bereich
- Bestimmung der Varianz oder Standardabweichung in jedem der Zeitintervalle anstatt Bildung des arithmetischen Mittels der Differenzbeträge
- Gewichtete Mittelungen, bspw. indem weiter vom Zeitpunkt tₓ entfernte Punkte stärker oder weniger stark berücksichtigt werden.
- In Schritt d) Auswahl eines anderen charakteristischen Vektors, bspw. durch Wahl eines bestimmten Messwerts anstelle eines Mittelwerts (unter der Annahme, dass bei der festgestellten geringen Messwertveränderung im Ruheabschnitt ein einzelner Messwert bereits repräsentativ ist)
- Auswahl eines mit dem Zeitintervall tₓ nicht identischen Zeitabschnitts als Ruheabschnitt. Beispielsweise kann ein nur einen zentralen Bereich des Zeitintervalls enthaltender Zeitabschnitt ausgewählt und in diesem der Durchschnittswert berechnet werden, insbesondere wenn die Zeitintervalle vergleichsweise gross gewählt werden. Andererseits kann auch ein grösserer, das Zeitintervall ganz enthaltender Bereich gewählt werden, beispielsweise wenn der Ruheabschnitt erwartungsgemäss in einem grösseren Zeitabschnitt verhältnismässiger Ruhe liegt.

Der Betrag des in diesem Verfahren als mittlerer Vektor eines ruhenden Vektorknäuels ermittelten Nullpunktvektors entspricht in der Praxis dem Unterschied zwischen dem gemessenen Potential in der Ruhephase und dem isoelektrischen Nullpunkt und der Nullpunktvektor kann deshalb als biologisch sinnvoll begründete Nullpunktkorrektur im Eichverfahren verwendet werden.

Die Eichung oder Nullpunkt-Korrektur kann auf verschiedene Weise vorgenommen werden, wie es dem Fachmann an sich bekannt ist. In einer Ausführungsform des erfindungsgemässen Verfahrens wird der Nullpunktvektor oder Referenzvektor vom Vektor aller Messwerte eines Herzzyklus oder der ganzen Messperiode subtrahiert. In anderen Ausführungsformen werden zuerst die Nullpunktvektoren mehrerer Herzschläge berechnet, dann gemittelt und dann der gemittelte Nullpunktvektor vom Vektor jedes Messwertes subtrahiert. Selbstverständlich kann die Nullpunktkorrektur auch graphisch ausgeführt und dargestellt werden. Beispielsweise können aufeinanderfolgende Nullpunktvektoren durch eine Gerade oder eine andere sinnvolle Verbindungskurve wie ein Spline oder eine numerisch ermittelte Näherungskurve verbunden werden.

Eine weitere vorteilhafte Verwendung der Nullpunktbestimmung ist die Berechnung des Z-Punktes. Als Z-Point (Zero-Point) bezeichnet man in der Kardiologie den Zeitpunkt, zu welchem die Kammer-Depolarisation beginnt. Er definiert demnach den Beginn der R-Welle oder R-Schlinge. Er ist nicht gleichzusetzen mit dem isoelektrischen Nullpunkt sondern liegt kurz danach und sein Potential sollte daher auch nicht auf 0 Volt Potential korrigiert werden. Der Z-point ist zeitlich da anzusiedeln, wo der Vektor räumlich das Knäuel, das die Ruhephase darstellt, endgültig verläßt, um die R-Schlinge zu durchlaufen. Er liegt demnach meist in der Nähe des isoelektrischen Nullpunktes und kann innerhalb oder ausserhalb des Ruheabschnittes liegen.

Nachdem der bestmöglichst dem isoelektrischen Nullpunkt entsprechende Nullpunkt gefunden wurde, kann auf ähnliche Weise auch leicht der Z-Point gefunden werden: Es wird ein zeitlicher Suchbereich definiert, in dem der Z-Point gefunden werden soll. Der Suchbereich muss gross genug sein, um den Z-Point zu beinhalten und erstreckt sich vom Zeitpunkt niedrigster mittlerer Geschwindigkeit des Vektors bis zu dem Punkt, wo der Betrag des Vektors sich um den Betrag Epsilon vom isoelektrischen Nullpunkt entfernt hat und vor dem R-Maximum nicht wieder näher an ihn herankommt. Der Betrag Epsilon kann in Microvolt definiert werden und sollte einen geeigneten niedrigen Wert haben.

Selbstverständlich können auf diese Art und Weise auch beliebige andere Punkte im Verlauf des Herzzyklus ermittelt werden, so lange sich eine Abhängigkeit eines solchen Punktes zum Nullpunkt darstellen lässt. Eine derartige Verwendung des erfindungsgemässen Verfahrens lässt sich selbstverständlich auch auf die Messung anderer biologischer Abläufe übertragen.

## Patentansprüche

1. Verfahren zur Verarbeitung von Messwerten einer diagnostischen Messvorrichtung, wobei die Messvorrichtung eine Reihe von Messwerten generiert, welche als n-dimensionale Vektoren darstellbar sind, wobei n einen Wert von mindestens 2 einnimmt, wobei das Verfahren durch eine Komponente der Messvorrichtung, die eine Programmierung aufweist, durchgeführt wird und der Eichung der Messvorrichtung dient, und im Verfahren ein Suchbereich definiert wird für einen in diesem Suchbereich enthaltenen Ruheabschnitt, wobei der Ruheabschnitt und ein in diesem Ruheabschnitt liegender mittlerer Vektor aus im Suchbereich gewonnenen Messwerten ermittelt werden, und wobei dieser mittlere Vektor als Referenzvektor zur Eichung der Messvorrichtung festgelegt wird, **dadurch gekennzeichnet, dass**
der Referenzvektor zur Eichung mit einem numerischen Verfahren ermittelt wird, welches in dem Suchbereich mit einer Anzahl ausgewählter Zeitpunkte mindestens die folgenden vier Schritte durchführt:
a) Berechnung des mittleren Vektors in einem Zeitabschnitt delta um jeden ausgewählten Zeitpunkt im Suchbereich;
b) Berechnung einer mittleren Abweichung aller Vektoren vom mittleren Vektor im Zeitabschnitt delta;
c) Identifikation des Ruheabschnitts als Zeitabschnitt delta mit der kleinsten mittleren Abweichung aller Vektoren vom mittleren Vektor;
d) Identifikation des Referenzvektors als der mittlere Vektor in dem als Ruheabschnitt identifizierten Zeitabschnitt delta.

2. Verfahren nach Anspruch 1, **dadurch gekennzeichnet, dass** sich die Messvorrichtung zur Aufzeichnung einer elektrischen Aktivität eines Herzmuskels eignet.

3. Verfahren nach Anspruch 2, **dadurch gekennzeichnet, dass** sich ein zeitlicher Suchbereich für den physiologisch begründeten Ruheabschnitt von einem Potentialmaximum einer Vorhoferregung bis zu einem Potentialmaximum einer Kammer-Depolarisation eines Herzzyklus erstreckt.

4. Verfahren nach einem der Ansprüche 2 oder 3, wobei der Referenzvektor zur Eichung der Messvorrichtung verwendet wird, indem der Referenzvektor von Vektoren aller Messwerte eines Herzzyklus oder einer ganzen Messperiode subtrahiert wird.

5. Verfahren nach einem der Ansprüche 2 bis 4, **dadurch gekennzeichnet, dass** zuerst die Nullpunktvektoren mehrerer Herzschläge berechnet, dann gemittelt und dann der gemittelte Nullpunktvektor vom Vektor jedes Messwertes eines Herzzyklus subtrahiert wird.

6. Verfahren nach Anspruch 1, **dadurch gekennzeichnet, dass** aus der Reihe der durch die diagnostische Messvorrichtung generierten Messwerte mindestens ein charakteristischer Messpunkt bestimmt wird, zu dessen Bestimmung der ermittelte Referenzvektor als ein Bezugspunkt dient, wobei der charakteristische Messpunkt ein Z-Punkt ist, wobei als Z-Punkt der erste nach dem Nullpunkt liegende Messwert definiert wird, dessen Betrag im Koordinatensystem mit dem Referenzvektor als Nullpunkt mindestens einem Schwellenwert Epsilon entspricht, welchen er nicht mehr unterschreitet.

7. Vorrichtung zur Messung von biologischen Signalen, **dadurch gekennzeichnet, dass** mindestens eine Komponente der Messvorrichtung eine Programmierung aufweist, um das Verfahren nach einem der Ansprüche 1-6 auszuführen.

8. Vorrichtung nach Anspruch 7 zur Messung von biologischen Signalen, wobei die Signale Potentiale sind, und die Vorrichtung mindestens zwei Elektroden aufweist sowie ein Gerät oder Gerätekomponenten zur Erfassung von Messwerten entsprechend Signalen dieser Elektroden, sowie zur Messwertverarbeitung und zur Darstellung der verarbeiteten Messwerte.

9. Vorrichtung nach Anspruch 8, **dadurch gekennzeichnet, dass** sie eine kardiographische oder kardiogoniometrische Vorrichtung ist.

## Claims

1. A method for calibrating a diagnostic measuring device, wherein the measuring device generates a sequence of measured values that can be represented as n-dimensional vectors, with n assuming a value of at least 2, wherein the method is performed by a component of the measuring device that comprises a programming and serves for the calibrating of the measuring device, and in the method a search region is defined for a rest segment contained in this search region, and wherein the rest segment and a mean vector lying in this rest segment are established from measured values obtained in the search region, and wherein this mean vector is set as reference vector for calibrating the measuring device, **characterised in that**
the reference vector for calibrating is established using a numerical method, which carries out at least the following four steps in the search region with a number of selected points in time:
a) calculating the mean vector in a time segment delta around each selected point in time in the search region;
b) calculating a mean deviation of all vectors from the mean vector in the time segment delta;
c) identifying the rest segment as time segment delta with the smallest mean deviation of all vectors from the mean vector;
d) identifying the reference vector as the mean vector in the time segment delta identified as rest segment.

2. Method as claimed in one claim 1, **characterised in that** the measuring device is suited for recording an electrical activity of a cardiac muscle.

3. Method as claimed in claim 2, **characterised in that** a temporal search region for the physiologically based rest segment extends from a maximum potential of an atrial excitation to a maximum potential of a ventricular depolarization of a cardiac cycle.

4. Method as claimed in one of claims 2 or 3, wherein the reference vector is used for calibrating the measuring device by the reference vector being subtracted from vectors of all measured values of a cardiac cycle or a whole measurement period.

5. Method as claimed in one of claims 2 to 4, **characterised in that** first the null point vectors of several heartbeats are determined, then averaged and then the average null point vector is subtracted from the vector of each measured value of a cardiac cycle.

6. Method as claimed in claim 1, **characterised in that** at least one characteristic measurement point is determined from the sequence of measured values generated by the diagnostic measuring device, with the established reference vector serving as a reference point for determining said characteristic measurement point, wherein the characteristic measurement point is a Z-point, wherein the Z-point is defined as the first measured value, lying after the null point, whose magnitude at least corresponds to a threshold epsilon in the coordinate system with the reference vector as null point and which no longer undershoots said threshold epsilon.

7. A device for measuring biological signals, **characterised in that** at least one component of the measuring device comprising a programming for carrying out the method as claimed in one of claims 1-6.

8. Device as claimed in claim 7 for measuring biological signals, wherein the signals are potentials and the device comprises at least two electrodes and an instrument or instrument components for capturing measured values corresponding to signals of these electrodes and also for processing measured values and for representing the processed measured values.

9. Device as claimed in claim 8, **characterised in that** it is a cardiographic or cardiogoniometric device.

## Revendications

1. Procédé de traitement de valeurs de mesure d'un ensemble de mesure de diagnostic, l'ensemble de mesure produisant une série de valeurs de mesure qui peuvent être représentées sous la forme de vecteurs à n dimensions, n ayant une valeur d'au moins 2, le procédé étant exécuté par un composant de l'ensemble de mesure qui présente une programmation et qui sert à étalonner l'ensemble de mesure,
une plage de recherche d'une section de repos contenue dans cette plage de recherche étant définie dans le procédé, la section de repos et un vecteur moyen situé dans cette section de repos étant déterminés à partir de valeurs de mesure obtenues dans la plage de recherche, ce vecteur moyen étant défini comme vecteur de référence pour l'étalonnage de l'ensemble de mesure,
**caractérisé en ce que**
le vecteur de référence servant à l'étalonnage est défini par un procédé numérique qui exécute au moins les quatre étapes ci-dessous en un certain nombre d'instants sélectionnés dans la plage de recherche :
a) calcul du vecteur moyen dans un intervalle de temps delta autour de chaque instant sélectionné dans la plage de recherche,
b) calcul de l'écart moyen de tous les vecteurs par rapport au vecteur moyen dans l'intervalle de temps delta,
c) définition de la section de repos comme intervalle de temps delta dans lequel l'écart moyen de tous les vecteurs par rapport au vecteur moyen est le plus petit,
d) définition du vecteur de référence comme vecteur moyen de l'intervalle de temps delta défini comme section de repos.

2. Procédé selon la revendication 1, **caractérisé en ce que** l'ensemble de mesure convient pour enregistrer l'activité électrique d'un muscle cardiaque.

3. Procédé selon la revendication 2, **caractérisé en ce que** la plage temporelle de recherche de la section de repos basée sur des critères physiologiques s'étend entre le potentiel maximum d'une excitation atriale et le potentiel maximum d'une dépolarisation des ventricules d'un cycle cardiaque.

4. Procédé selon l'une des revendications 2 ou 3, dans lequel le vecteur de référence est utilisé pour étalonner l'ensemble de mesure en soustrayant le vecteur de référence des vecteurs de toutes les valeurs de mesure d'un cycle cardiaque ou d'une période entière de mesure.

5. Procédé selon l'une des revendications 2 à 4, **caractérisé en ce que** tous les vecteurs de point zéro de plusieurs battements cardiaques sont calculés, leur moyenne est formée et le vecteur moyen de point zéro est soustrait du vecteur de chaque valeur de mesure d'un cycle cardiaque.

6. Procédé selon la revendication 1, **caractérisé en ce qu'**au moins un point de mesure caractéristique est défini à partir de la série des valeurs de mesure produites par l'ensemble de mesure de diagnostic, le vecteur de référence déterminé pour cette définition servant de point de référence, le point de mesure caractéristique étant un point Z, la première valeur de mesure située après le point zéro étant définie comme point Z dont le niveau dans le système de coordonnées qui présente le vecteur de référence comme point zéro correspond à au moins une valeur de seuil epsilon à laquelle il n'est plus inférieur.

7. Ensemble de mesure de signaux biologiques, **caractérisé en ce qu'**au moins un composant de l'ensemble de mesure présente une programmation permettant de mettre en oeuvre le procédé selon l'une des revendications 1 à 6.

8. Ensemble selon la revendication 7 pour la mesure de signaux biologiques, dans lequel les signaux sont des potentiels et l'ensemble présente au moins deux électrodes ainsi qu'un appareil et les composants d'appareil permettant de saisir sur ces électrodes des signaux correspondant à des valeurs de mesure, de traiter les valeurs de mesure et de présenter les valeurs de mesure traitées.

9. Ensemble selon la revendication 8, **caractérisé en ce que** c'est un ensemble de cardiographie ou de cardiogoniométrie.
